# EUROPEAN PATENT APPLICATION

(11) **EP 4 578 432 A1**
(43) Date of publication of application: **02.07.2025**
(21) Application number: 23856767.1
(22) Date of filing: 22.08.2023
(51) Int. Cl.: A61F 13/08

(54) **SOCK FOR TREATING TIBIA PAIN**

(30) Priority: 25.08.2022 ES 202231401 U
(71) Applicant: Fixtoe Device, S.L., 03550 San Juan de Alicante Alicante (ES)
(72) Inventor: LUCAS PICAZO, David, 03550 Alicante (ES); MONZÓ PÉREZ, Francisco, 03550 Alicante (ES)
(74) Representative: Ibarra Garcia, Isabel
(86) International application number: PCT/ES2023/070523
(87) International publication number: WO 2024/042259

(57) **Abstract**

The invention discloses a sock for treating tibia pain that is made up of three parts: a first part having a thickness between 1 and 4 mm that covers the plantar region of the big toe, a second part having a maximum thickness of 6 mm that covers the metatarsal plantar region, from the first to the third metatarsal, extending from the sulcus of the toes, and a third part having a maximum thickness of 6 mm that covers at most the inner 2/3 of the plantar region of the heel. Advantageously, the defined configuration helps to reduce painful symptoms associated with the pathology of tibial periostitis, medial tibial stress syndrome and venous return, thus improving blood circulation in the lower limbs and the feeling of heaviness in daily activities and sports.

## Description

### OBJECT OF THE INVENTION

The present invention relates to a sock for treating tibia pain, specifically, for treating pain caused by tibial periostitis or medial tibial stress syndrome. The sock of the invention is intended to conservatively address tibial periostitis or other mechanical causes responsible for pain in the middle and distal third and anteromedial aspect of the tibia.

Advantageously, the sock of the invention is easy for the patient to put on and provides high effectiveness and durability.

### BACKGROUND OF THE INVENTION

As is known, tibial periostitis or medial tibial stress syndrome is one of the pathologies with the highest incidence in the adult world population in the field of sports, and is the main cause of mechanical pain in the middle third and distal third of the tibia, as well as in the anteromedial aspect or area of the tibia, which pain occurs when and after practicing sports, such as running or sports that involve jumping. In the field of sports podiatry, it is one of the most common lower limb injuries among athletes such as runners or soldiers.

From an anatomical point of view, the middle and distal third of the tibia has a smaller cross-section area (i.e., a narrower cylindrical bone), making it a structure that is biomechanically more vulnerable to the action of valgus bending forces. Moreover, there are also various musculotendinous structures that have their anatomical trajectories and insertions in said area of the tibia, and may be responsible for generating a deforming tensile stress on the bone structure. Biomechanically, excessive bone stress on the middle and distal third of the tibia is related to various internal and external pathological factors of mechanical origin and can boil down to the presence of valgus bending forces on the tibia, as well as the presence of tensile forces originating in the perilesional musculature such as the soleus muscle, the posterior tibial muscle and the common flexor longus muscle of the toes.

Tibial periostitis or tibial pain is frequently observed in patients having deformities such as tibiae and/or bowlegs, inverted forefoot, pronated feet, etc. In the field of sports podiatry, it is one of the most common lower limb injuries among athletes such as runners or soldiers.

The following internal and external factors should be highlighted among the multiple causes that can lead to tissue damage in the middle and distal third of the tibia:
- Pronated feet and/or flat feet (feet with medial deviations of the subtalar rotation axis).
- Previous history of tibial stress fracture.
- Shortening of the gastrocnemius musculature (gastrocnemius equinus).
- Weakness or atrophy of the musculature of the anterolateral compartment such as the tibialis anterior and peroneals.
- Tibia vara.
- Bowlegs.
- Rigid inverted forefoot deformity.
- Incorrect training plans.
- Improper use of sports shoes.
- Improper use of training surfaces.
- Unsuitable sport-based movement.
- Alterations in bone metabolism. Osteopenia.
- Dysmenorrhea and/or amenorrhea.
- Vitamin D and E deficiency.
- Tibial stress fracture.
- Chronic anterior compartment syndrome.
- Fracture of the tibiofibular syndesmosis.
- Deep vein thrombosis.
- Nerve entrapment.

Clinically, the main symptoms of tibial periostitis or medial tibial stress syndrome are as follows:
- Pain in the anterior and anteromedial area of the middle-distal third of the tibia.
- Mechanical pain that increases with increased activity and worsens at the end of the day.
- Morning pain and stiffness.
- In early stages there is no pain while resting, while in advanced stages it is accompanied by pain while resting or walking.
- Stress fracture phenomenon.
- Pain when digitopressure is performed on the tibial periosteum.
- Pain that improves with rest, taking NSAIDs (non-steroidal anti-inflammatory drugs), cryotherapy, massaging and stretching of the musculature of the sole of the foot and gastrocnemius musculature, use of Low Dye Tape-type functional bandages associated with wedge-shaped felts on the medial plantar heel and medial plantar forefoot and customised orthopaedic insoles with medial heel skive and forefoot supination wedges, etc.
- Pain that worsens with increasing stride length.
- Pain that improves by reducing stride length and promoting midfoot and forefoot support.
- Inability to walk barefoot or train on hard surfaces.

Currently, when diagnosing a process of tibial periostitis or medial tibial stress syndrome, conservative treatment measures are applied as the first therapeutic choice, in order to, on one hand, reduce the painful inflammatory phase, and on the other hand, control the biomechanical factors responsible for the excessive bone stress on the tibia and tensile stress on the musculotendinous structures originating from and covering the damaged tibial area, such as the soleus muscle, the common flexor longus muscle of the toes and the posterior tibial muscle.

Among the conservative orthopaedic treatment alternatives, Low Dye Tape-type adhesive bandages combined with wedges made of a felt material are known as a short-term treatment, these bandages being placed in the plantar and medial area of the heel, covering the inner 2/3 of the heel, and in the plantar and medial area of the forefoot, covering the area comprised between the first metatarsal to the third or fourth metatarsal. Customised orthopaedic insoles with medial heel skive and forefoot supination wedge associated with suitable footwear are known as a medium and long-term treatment.

There is ample scientific evidence on the positive effect shown by the Low Dye Tape-type bandage associated with felt wedges as a first-choice treatment (4-6 weeks of initial treatment) on the control or reduction of pain reported by patients with tibial periostitis, showing itself as one of the most successful and effective short-term treatment alternatives, either alone and/or as an aid to treatment with customised orthopaedic insole. In this case, the design of the orthopaedic insole will be aimed at controlling bone stress on the tibia and tensile stress on the musculotendinous structures originating from and covering the damaged tibial area such as the soleus muscle, the common flexor longus muscle of the toes and the posterior tibial muscle by means of an internal arch support, a part made of medium-density EVA (ethylene vinyl acetate) in the form of a medial heel skive-type rearfoot supination wedge having a thickness of 8-10 mm and covering the plantar and medial area of the heel, about the inner 2/3 thereof, and a part made of medium-density EVA in the form of forefoot supination wedge having a thickness of 3-5 mm and covering the plantar area from the first metatarsal to the fourth metatarsal.

However, the use of the bandage associated with adhesive felt or EVA wedges has the drawbacks of a troublesome daily hygiene, the need to change the bandage every 2-3 days which cannot be performed by the patient him/herself and depends on the help of a clinical professional, damage to skin, such as contact dermatitis processes, etc.

The use of the Low Dye Tape bandage associated with felt wedges significantly improves the symptoms or pain in tibial periostitis, but is accompanied by a decrease in the quality of daily life during daily activities.

In addition, treatments with adhesive bandages prevent the skin from breathing and are very uncomfortable.

Based on the foregoing, the applicant of the present utility model understands the need to provide a device that translates into the creation of a technical sock which satisfactorily solves the preceding problems, facilitating patient's autonomy and hygiene.

### DESCRIPTION OF THE INVENTION

The sock proposed for treating tibia pain, mainly caused by tibial periostitis or medial tibial stress syndrome pain, overcomes in a fully satisfactory manner the problems set forth above based on a simple and effective solution.

Therefore, the sock of the invention provides a treatment that is an alternative to Low Dye Tape-type bandage associated with felt wedges and allows solving the presence of painful symptoms on the leg and/or tibia or any other cause of mechanical tibia pain.

According to the essence of the invention, the sock for treating tibia pain is formed by a main body made of an elastic and compressive fabric, with a thickness between 4 and 12 mm, and, as is typical in this type of garment, the main body has a leg, a plantar surface, i.e., the lower surface coinciding with the sole of the user's foot, and a toe to accommodate the toes. Optionally, the toe is divided into at least two compartments, such that a first compartment is arranged so as to coincide with the big toe for its accommodation and the second compartment allows the accommodation of the rest of the toes together.

Preferably, the elastic and compressive fabric forming the main body of the sock is a washable and breathable bamboo, polyester, elastane and/or nylon fabric. However, the present invention is not limited to these materials, and the sock can be made of any fabric with elastic and compressive fibres that promotes breathability and can be washed.

At least three parts are arranged on the plantar surface of the sock for treating tibia pain:
- a first part having a thickness between 1 and 4 mm, preferably 3 mm, that covers the plantar region of the big toe,
- a second part having a maximum thickness of 6 mm, preferably 5 mm, that covers the metatarsal plantar region, from at least the first to the third metatarsal, extending from the sulcus of the toes, and
- a third part having a maximum thickness of 6 mm, preferably 5 mm, that covers at most the inner 2/3 of the plantar region of the heel, to increase the weight of the ground on the medial plantar calcaneus, thereby generating a supination force on the foot.

Preferably, the first part, the second part and the third part arranged along the plantar surface of the sock have a hardness of between 35º and 45º Shore A.

It should be noted that Shore hardness is a scale for measuring the elastic hardness of materials, determined from the elastic reaction of the material when an object is dropped on it, the A scale being used for tires, rubber bands, etc.

Optionally, the second part and the third part provide a wedge shape of decreasing thickness, such that at their outer end, the parts have a thickness of 5 mm that decreases towards their inner end. Advantageously, the wedge shape of the part allows the reactive force or pressure of the ground on the user's foot to be distributed progressively and more evenly.

Moreover, the main body of the sock can be configured to provide all types of legs: high, medium and low, adapting to the needs and morphology of each user, with the leg preferably extending to a maximum height of 1 cm below the user's kneecap.

Optionally, the main body of the sock is provided with an enveloping portion in correspondence with the instep and the plantar arch of the sock, wherein the enveloping portion provides a tension greater than the tension provided by the elastic and compressive fabric forming the rest of the main body. In that sense, the enveloping portion helps stabilize the internal arch of the foot. In one embodiment of the invention, the enveloping portion has fibres or strips arranged longitudinally and/or transversely with respect to the surface of the sock providing the mentioned tension that is greater than the tension of the rest of the main body of the sock.

In an alternative embodiment, the plantar surface region of the sock coinciding with the internal arch of the foot is provided with a plurality of wavy longitudinal reliefs which provide a tension greater than the tension provided by the elastic and compressive fabric forming the rest of the main body. Preferably, the longitudinal reliefs are made of the same fabric as the main body, but with a higher degree of tension, like a spring, providing more stability to the longitudinal arch of the foot and controlling its flattening.

Advantageously, the sock of the invention provides the patient with stability, support, comfort and rest throughout the leg and foot.

Additionally, the sock of the invention provides the following advantages:
- Easy to put on and use, with a longer average life than known treatment alternatives.
- It promotes the reduction of painful symptoms associated with the pathology of tibial periostitis, medial tibial stress syndrome or another cause related to tibia pain.
- It promotes venous return, thus improving blood circulation in the lower limbs and the sensation of heaviness during daily activities and sports.
- It prevents skin irritations as it is made of a breathable and washable material (unlike the bandage systems).

### DESCRIPTION OF THE DRAWINGS

To complement the description that will be made below and in order to help better understand the features of the invention according to two preferred embodiments thereof, there is attached as an integral part of said description a set of drawings wherein the following is depicted in an illustrative and non-limiting manner:
Figure 1 shows a bottom view of the sock for treating tibia pain according to a first preferred embodiment of the invention, in which the sock has an enveloping portion that provides a tension greater than that of the rest of the fabric of the sock, and in which the patient's foot inside the sock is depicted with a dotted line.
Figure 2 shows a side view of the sock for treating tibia pain from the previous figure placed on a patient's foot, such that the inner side of the foot is shown.
Figure 3 shows a front view of the sock for treating tibia pain from the previous figures placed on a patient's foot, in which the detail of the wedge shape of the second part can be seen with the toes lifted.
Figure 4 shows a front view of the sock for treating tibia pain from the previous figures placed on a patient's foot, in which the detail of the wedge shape of the second part can be seen.
Figure 5 shows a front perspective view of the sock for treating tibia pain from the previous figures placed on a patient's foot, in which the detail of the toe with two compartments can be seen.
Figure 6 shows a rear perspective view of the sock for treating tibia pain from the previous figures placed on a patient's foot, in which the detail of the third part and the outer side of the foot can be seen.
Figure 7 shows a bottom view of the sock for treating tibia pain according to a second preferred embodiment of the invention, in which the plantar surface of the sock is provided with a plurality of wavy longitudinal reliefs which provide a tension greater than that of the rest of the fabric of the sock.

### PREFERRED EMBODIMENT OF THE INVENTION

In view of the mentioned figures, it can be seen that according to two preferred embodiments of the invention, the sock for treating tibia pain starts from the conventional structuring of a sock having a main body (4) in which a leg, a plantar surface and a toe for accommodating the toes are defined.

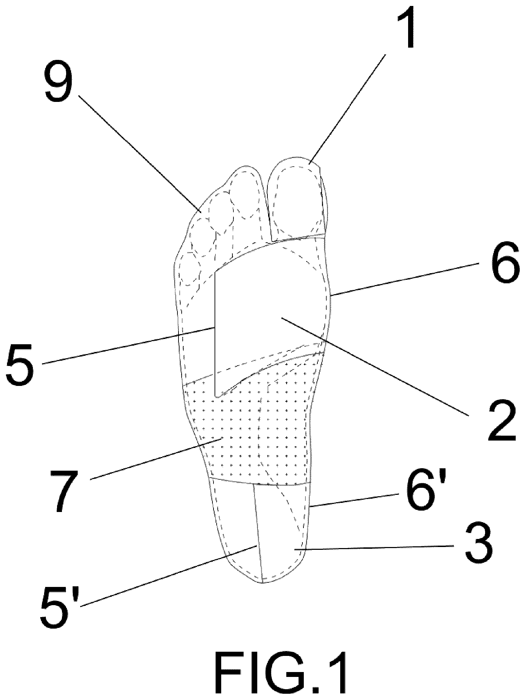

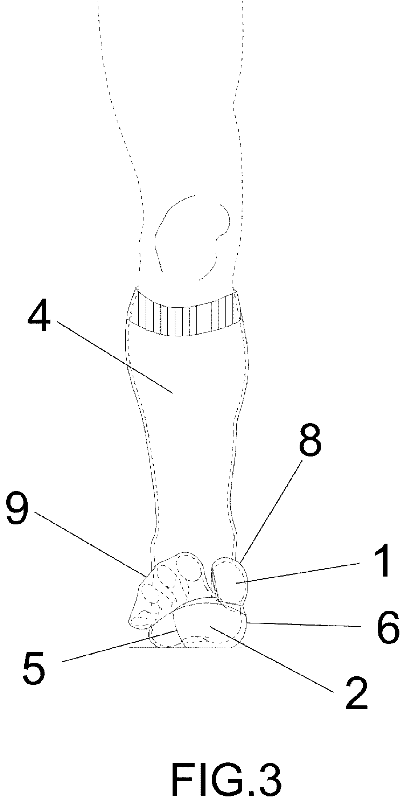

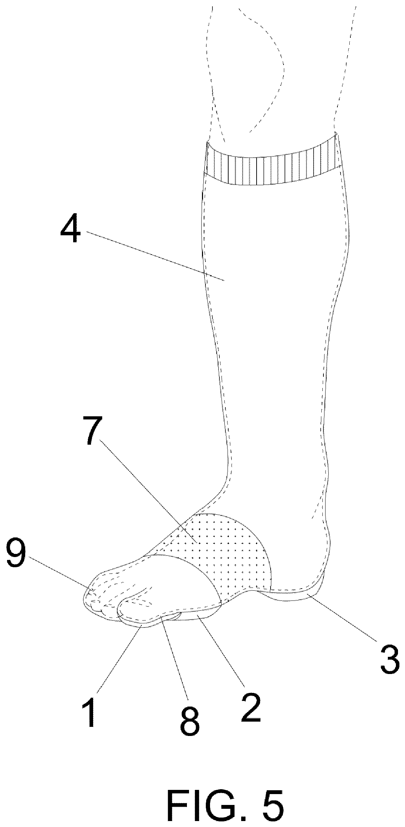

As can be seen in Figures 1, 3, 5 and 7, the toe is divided into a first compartment (8) for accommodating the big toe and a second compartment (9) for accommodating the rest of the toes, in order to facilitate the correct support of the toes on the different structures or parts of the sock of the invention.

As can be seen in Figure 1, in a preferred embodiment of the invention three parts are arranged on the plantar surface of the sock:
- a first part (1) having a thickness between 1 and 4 mm that covers the plantar region of the big toe,
- a second part (2) that covers the metatarsal plantar region, from at least the first to the third metatarsal, extending from the sulcus of the toes, said part (2) having a wedge shape of decreasing thickness, such that at its outer end (6), the second part (2) has a thickness of 5 mm that decreases towards its inner end (5), and
- a third part (3) having a wedge shape of decreasing thickness, such that at its outer end (6'), the third part (3) has a thickness of 5 mm that decreases towards its inner end (5'), such that the third part (3) covers at most the inner 2/3 of the plantar region of the heel.

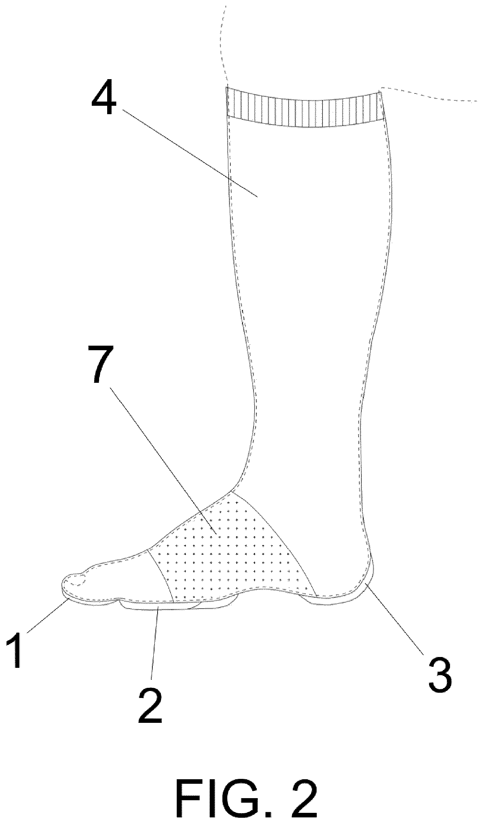

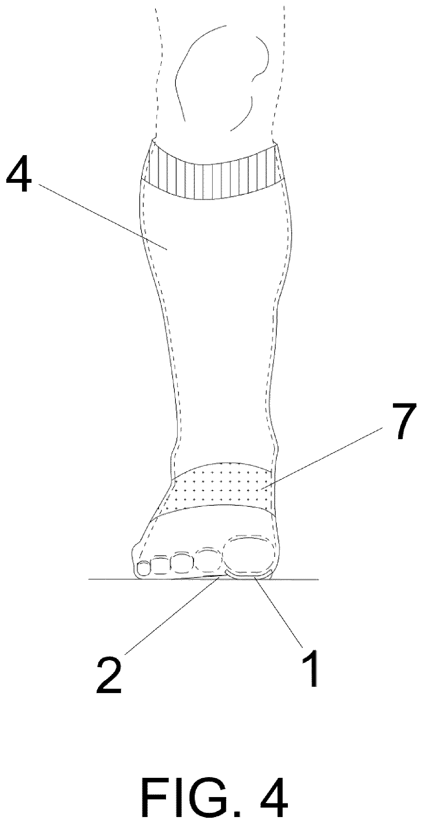

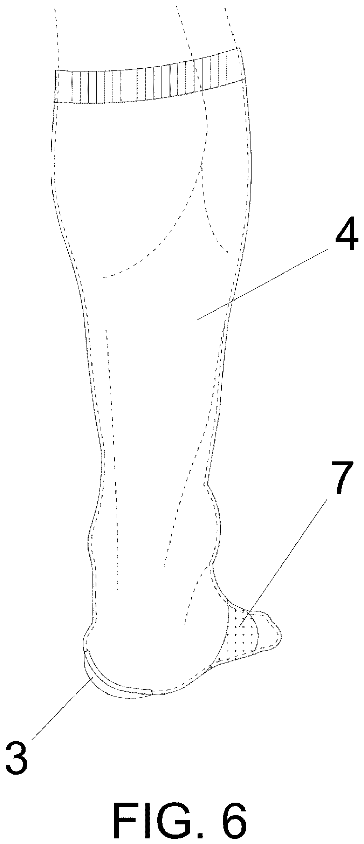

Additionally, as can be seen in Figures 1, 2, 4, 5 and 6, the main body (4) is provided with an enveloping portion (7) in correspondence with the instep and the plantar arch of the sock, wherein the enveloping portion (7) provides a tension greater than the tension provided by the elastic and compressive fabric forming the rest of the main body (4).

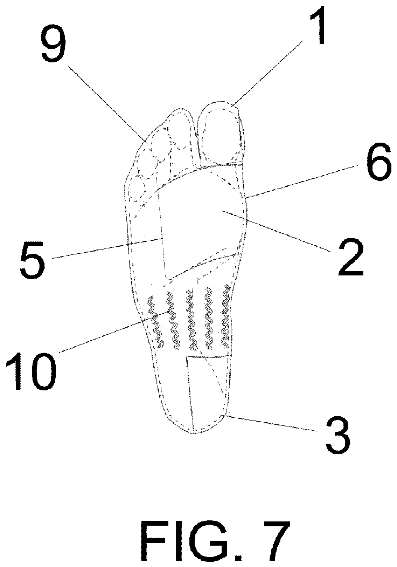

Figure 7 depicts the sock for treating tibia pain according to a second preferred embodiment of the invention, such that the plantar surface region of the sock coinciding with the internal arch of the foot is provided with a plurality of wavy longitudinal reliefs (10), wherein the longitudinal reliefs (10) provide a tension greater than the tension provided by the elastic and compressive fabric forming the rest of the main body (4).

The sock for treating tibia pain according to the two non-limiting preferred embodiments described provides a therapeutic element with strategically distributed support areas that allow relief for tibial periostitis, medial tibial stress syndrome or other causes responsible for mechanical pain in the anteromedial aspect or area of the tibia by means of an effective and easy-to-use device, providing autonomy to the patient, and without unwanted side effects such as skin irritation.

## Claims

1. A sock for treating tibia pain of the types having a main body (4) made of an elastic and compressive fabric with a thickness between 4 mm and 12 mm, in which there are defined a leg, a plantar surface and a toe for accommodating the toes, **characterised in that** at least three parts are arranged on the plantar surface of the sock:
- a first part (1) having a thickness between 1 and 4 mm that covers the plantar region of the big toe,
- a second part (2) having a maximum thickness of 6 mm that covers the metatarsal plantar region, from at least the first to the third metatarsal, extending from the sulcus of the toes, and
- a third part (3) having a maximum thickness of 6 mm that covers at most the inner 2/3 of the plantar region of the heel.

2. The sock for treating tibia pain according to claim 1, **characterised in that** the first part (1), the second part (2) and the third part (3) have a hardness between 35º and 45º Shore A.

3. The sock for treating tibia pain according to claim 1, **characterised in that** the second part (2) provides a wedge shape of decreasing thickness, such that at its outer end (6), the second part (2) has a thickness of 5 mm that decreases towards its inner end (5).

4. The sock for treating tibia pain according to claim 1, **characterised in that** the third part (3) provides a wedge shape of decreasing thickness, such that at its outer end (6'), the third part (3) has a thickness of 5 mm that decreases towards its inner end (5').

5. The sock for treating tibia pain according to claim 1, **characterised in that** the main body (4) defines a leg extending to a maximum height of 1 cm below the user's kneecap.

6. The sock for treating tibia pain according to claim 1, **characterised in that** the elastic and compressive fabric is a bamboo, polyester, elastane and/or nylon fabric.

7. The sock for treating tibia pain according to claim 1, **characterised in that** the main body (4) is provided with an enveloping portion (7) in correspondence with the instep and the plantar arch of the sock, wherein the enveloping portion (7) provides a tension greater than the tension provided by the elastic and compressive fabric forming the rest of the main body (4).

8. The sock for treating tibia pain according to claim 1, **characterised in that** the plantar surface region of the sock coinciding with the internal arch of the foot is provided with a plurality of wavy longitudinal reliefs (10), wherein the longitudinal reliefs (10) provide a tension greater than the tension provided by the elastic and compressive fabric forming the rest of the main body (4).

9. The sock for treating tibia pain according to claim 8, **characterised in that** the longitudinal reliefs (10) are made of the same fabric as the main body (4).

10. The sock for treating tibia pain according to any of the preceding claims, **characterised in that** the toe is divided into at least two compartments, wherein the first compartment (8) allows the accommodation of the big toe and the second compartment (9) allows the accommodation of the rest of the toes.
